(19) Europäisches Patentamt
European Patent Office
Office européen des brevets

(11) **EP 3 581 652 A1**

(12) **EUROPEAN PATENT APPLICATION**
published in accordance with Art. 153(4) EPC

(43) Date of publication:
**18.12.2019 Bulletin 2019/51**

(51) Int Cl.:
*C12N 15/09* (2006.01)   *C12Q 1/68* (2018.01)

(21) Application number: **18750659.7**

(22) Date of filing: **09.02.2018**

(86) International application number:
**PCT/JP2018/004703**

(87) International publication number:
**WO 2018/147438 (16.08.2018 Gazette 2018/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **13.02.2017   JP 2017024397**

(71) Applicant: **Kyoto University**
**Kyoto-shi, Kyoto 606-8501 (JP)**

(72) Inventors:
• **MATSUDA, Fumihiko**
**Kyoto-shi**
**Kyoto 606-8501 (JP)**

• **KAWAGUCHI, Shuji**
**Kyoto-shi**
**Kyoto 606-8501 (JP)**
• **SHIMIZU, Masakazu**
**Kyoto-shi**
**Kyoto 606-8501 (JP)**
• **HIGASA, Koichiro**
**Kyoto-shi**
**Kyoto 606-8501 (JP)**

(74) Representative: **Hoffmann Eitle**
**Patent- und Rechtsanwälte PartmbB**
**Arabellastraße 30**
**81925 München (DE)**

(54) **PCR PRIMER SET FOR HLA GENE, AND SEQUENCING METHOD USING SAME**

(57)   The present invention provides an HLA gene amplification primer set containing a primer consisting of each base sequence shown in SEQ ID NO: 1 - 16. It also provides a highly efficient and highly uniform sequencing method using the primer set.

**Fig. 7**

EP 3 581 652 A1

**Description**

[Technical Field]

The present invention relates to a PCR primer set for classical HLA gene, and a highly efficient and highly uniform sequencing method using same.

[Background Art]

Human Leukocyte Antigen (HLA) which is a major histocompatibility complex (MHC) of human is an important protein relating to immune responses and deeply involved in diseases related to immunity. The gene region encoding HLA is located at human chromosome 6 short arm part 6p21.3, and important genes of HLA include HLA-A, HLA-B, HLA-C belonging to class I molecule expressed in almost all cells, and HLA-DRB1, HLA-DQB1, HLA-DPB1 belonging to class II molecule mainly expressed in the cells in the immune system. These HLA genes are deeply involved in tissue compatibility in organ transplantation and accurate determination (typing) of HLA allele is clinically extremely important. However, the above-mentioned 6 kinds of HLA genes are regions abundantly polymorphic among human genomes, with more than 10,000 kinds of alleles, and a typing method has not been established yet.

Hosomichi et al. produced primers that specifically anneal to the upstream region and the downstream region of each of the above-mentioned 6 kinds of HLA genes and reported a method for determining the base sequence of HLA gene by a long PCR method using the primers and a next-generation sequencer (NGS) (patent document 1, non-patent document 1). However, in a preliminary experiment performed by the present inventors with the subject being Japanese people, a problem was found that uniform amplification cannot be achieved because long PCR using the above-mentioned primers showed high amplification efficiency for HLA-C and extremely low amplification efficiency for HLA-DRB1. Particularly, in HLA-DRB1, a certain kind of allele was hardly amplified and genes other than the target such as HLA-DRB3 and the like were amplified. A similar problem has been reported not only when the subject is Japanese but also Asian, Caucasian or Black (non-patent document 2).

It has been reported that, to solve the problem of the above-mentioned method, Ehrenberg et al. performed PCR by adding one kind of primer (in the cases of HLA-A and HLA-C) or 3 kinds of primers (in the case of HLA-DRB1) to the above-mentioned primers (non-patent document 2). However, uniform amplification is influenced by the different number of primers for each gene in the aforementioned PCR. Furthermore, as regards HLA-DRB1, the primer sequences are completely the same for genes other than the target genes and thus the unintended genes may be amplified.

As other approach to HLA gene typing, a method including designing, for the above-mentioned 6 kinds of HLA genes, 120-base probes corresponding to various sequences of HLA alleles based on the database, and reading the genome fragments obtained by hybridizing cDNA and the probe by NGS has been reported (non-patent document 3). In this method, however, as many as 10,000 kinds of probes have been designed to cover as many sequences as possible of HLA alleles registered in the database, which in turn causes a problem of decreased amplification uniformity and collection efficiency. In addition, it is difficult to detect alleles other than the covered HLA alleles and a genome fragment of a gene other than the target genes may be mixed.

[Document List]

[Patent document]

patent document 1: JP-A-2016-10318

[non-patent documents]

    non-patent document 1: Hosomichi, K. et al., BMC Genomics, 14:355, 2013.
    non-patent document 2: Ehrenberg, P.K. et al., BMC Genomics, 15:864, 2014.
    non-patent document 3: Wittig, M. et al., Nucleic Acids Res., 43 (11) : e70, 2015

[SUMMARY OF THE INVENTION]

[Problems to be Solved by the Invention]

The present invention aims to provide a primer set enabling uniform amplification of HLA genes and capable of increasing the number of samples that can be processed at one time. In addition, the present invention aims to provide a highly efficient and highly uniform sequencing method using the primer set.

[Means of Solving the Problems]

Designing of PCR primer aiming at amplification of HLA gene has the following two contradictory problems.

A. Sequences of primers that can encompass as many kinds of HLA alleles as possible are selected.
B. For uniform amplification, a single primer is prepared for each amplification target region. When the regions overlap, the overlapping region should be as short as possible. Genes other than the target genes should not be amplified.

Therefore, the development of a primer set that solves the above-mentioned two problems (namely, fulfilling the above-mentioned conditions A, B) has been desired. To increase condition A, the number of primers needs to be increased or a sequence with high homology needs to be selected. In this event, condition B is difficult to achieve. The present inventor had an idea that a primer set fulfilling the two conditions of conditions A and condition B may be developed by performing the following steps 1 - 4.

1. Assembling sequence analysis data by next-generation sequencing of 6 genes in the samples of 768 Japanese people while eliminating reads derived from other genes.
2. Aligning the assembled results in all samples for each gene.
3. Regions having sequences common to all samples are detected from the results of 2 and noted as primer candidates.
4. Sequences having a sequence not common to other HLA genes and suitable for primers are selected from among the candidates of 3.

The present inventors have made further studies based on the conception of the above-mentioned idea and completed the present invention.
Accordingly, the present invention provides the following.

[1] A primer set for HLA-A gene amplification comprising a primer consisting of the base sequence shown in SEQ ID NO: 1 and a primer consisting of the base sequence shown in SEQ ID NO: 2.
[2] A primer set for HLA-B gene amplification comprising a primer consisting of the base sequence shown in SEQ ID NO: 3 and a primer consisting of the base sequence shown in SEQ ID NO: 4.
[3] A primer set for HLA-C gene amplification comprising a primer consisting of the base sequence shown in SEQ ID NO: 5 and a primer consisting of the base sequence shown in SEQ ID NO: 6.
[4] A primer set for HLA-DRB1 gene amplification comprising a primer consisting of the base sequence shown in SEQ ID NO: 7, a primer consisting of the base sequence shown in SEQ ID NO: 8, a primer consisting of the base sequence shown in SEQ ID NO: 9, and a primer consisting of the base sequence shown in SEQ ID NO: 10.
[5] A primer set for HLA-DQB1 gene amplification comprising a primer consisting of the base sequence shown in SEQ ID NO: 11 and a primer consisting of the base sequence shown in SEQ ID NO: 12.
[6] A primer set for HLA-DPB1 gene amplification comprising a primer consisting of the base sequence shown in SEQ ID NO: 13, a primer consisting of the base sequence shown in SEQ ID NO: 14, a primer consisting of the base sequence shown in SEQ ID NO: 15, and a primer consisting of the base sequence shown in SEQ ID NO: 16.
[7] A primer set for HLA gene amplification comprising not less than two selected from the group consisting of the primer set of [1], the primer set of [2], the primer set of [3], the primer set of [4], the primer set of [5], and the primer set of [6].
[8] A primer set for HLA gene amplification comprising all primer sets of [1] to [6].
[9] A method of sequencing an HLA gene comprising using the primer set of any of [1] to [8].
[10] A method of typing an HLA gene comprising using base sequence information obtained by the method of [9].
[11] A kit for HLA gene amplification comprising the primer set of any of [1] to [8].

[Effect of the Invention]

The primer set of the present invention is obtained by assembling and aligning the results of large-scale sequencing and designing based on the common sequences. Thus, using the primer set of the present invention, many of HLA alleles that were difficult to amplify by the conventional method can be amplified without omission. In the primer set of the present invention, primers having no homology with other genes are selected from huge sequence candidates by a computational approach. Thus, using the primer set of the present invention, non-specific amplification can be prevented. Therefore, misdetermination of allele due to contamination by unintended genes can be reduced, which contributes to allele determination with high accuracy. Furthermore, since a single primer is used for the object gene region, uniform PCR amplification is possible, the number of samples that can be processed at one time can be increased, and HLA typing cost can be reduced.

[Brief Description of the Drawings]

Fig. 1 shows a schematic diagram showing the assembly results of large-scale sequencing in the HLA-A gene in the present invention and the positions of primers for amplification and an outline of the primers.
Fig. 2 shows a schematic diagram showing the assembly results of large-scale sequencing in the HLA-B gene in the present invention and the positions of primers for amplification and an outline of the primers.
Fig. 3 shows a schematic diagram showing the assembly results of large-scale sequencing in the HLA-C gene in the present invention and the positions of primers for amplification and an outline of the primers.
Fig. 4 shows a schematic diagram showing the assembly results of large-scale sequencing in the HLA-DRB1 gene in the present invention and the positions of primers for amplification and an outline of the primers.
Fig. 5 shows a schematic diagram showing the assembly results of large-scale sequencing in the HLA-DQB1 gene in the present invention and the positions of primers for amplification and an outline of the primers.
Fig. 6 shows a schematic diagram showing the assembly results of large-scale sequencing in the HLA-DPB1 gene in the present invention and the positions of primers for amplification and an outline of the primers.
Fig. 7 shows analysis of the results of the coverage average in the sequencing results by MiSeq using the primer set of the present invention and the primer set of a previous report.

[Description of Embodiments]

The present invention provides a primer set capable of uniformly amplifying gene regions of HLA-A, HLA-B, HLA-C, HLA-DRB1, HLA-DQB1 or HLA-DPB1 of HLA gene (including translational region, intron region and a part of untranslated region on 5'-side and 3'-side) by PCR (hereinafter to be abbreviated as "the primer set of the present invention"). Also, the present invention provides a typing method of HLA gene using the primer set of the present invention.
In the present invention, a "primer set" means a combination of not less than two PCR primers capable of amplifying a given region of each HLA gene. A primer set consisting of two primers of a forward primer and a reverse primer corresponding to each region is sometimes referred to as a primer pair.
To be specific, the present invention provides at least one primer pair selected from the primer sets recited in the following Table 1.

[Table 1]

| primer | HLA | forward (F)/ reverse (R) | primer sequence (5'→3') | SEQ ID NO: | predicted length (Kb) |
|---|---|---|---|---|---|
| Kyoto_HLA_A_F | A | F | TTTCCAGAGAAGCCAATCAGTGTC | 1 | 3.1 |
| Kyoto_HLA_A_R | | R | ACAGACACATGCAGGTGCCTTTGCAGAAAC | 2 | |
| Kyoto_HLA_B_F | B | F | GTCGGGTCCTTCTTCCAGGATACTCG | 3 | 3.1 |
| Kyoto_HLA_B_R | | R | TCTTATTTTCAGTAGGGAAGTAAGAAGTTG | 4 | |
| Kyoto_HLA_C_F | C | F | GTCGGGTCCTTCTTCCTGAATACTCA | 5 | 3.2 |
| Kyoto_HLA_C_R | | R | CCACCTCCTCACATTATGCTAACAGGAACG | 6 | |

(continued)

| primer | HLA | forward (F)/ reverse (R) | primer sequence (5'→3') | SEQ ID NO: | predicted length (Kb) |
|---|---|---|---|---|---|
| Kyoto_HLA_DRB1_FF | DRB1 | F | CCCTCCATCTCCTTTACTCC | 7 | 6.3 - 11.3 |
| Kyoto_HLA_DRB1_FR | | R | TGCCTCCCCTCCCACAACAG | 8 | |
| Kyoto_HLA_DRB1_RF | | F | AGGAGGACCTGTGAACCAGAG | 9 | 7.5 - 10.7 |
| Kyoto_HLA_DRB1_RR | | R | TTTGAGAAACATTTAATAATGTAATGTGTTTGT | 10 | |
| Kyoto_HLA_DQB1_F | DQB1 | F | GTCCTTCAGCTCCAGTGCTGATTGGTTC | 11 | 6.7 |
| Kyoto_HLA_DQB1_R | | R | GATGAAAGGAGATGACCTGGTGG | 12 | |
| Kyoto_HLA_DPB1_FF | DPB1 | F | CTTATCTGACTGGTTAAAATGAGTATCACT | 13 | 6.0 |
| Kyoto_HLA_DPB1_FR | | R | GAACCCAAGACAAGTAAAATCCATCTC | 14 | |
| Kyoto_HLA_DPB1_RF | | F | CAGAGTCTTTCTTATACCAAAGTTGAAGAA | 15 | 8.2 |
| Kyoto_HLA_DPB1_RR | | R | GAGGATTTCATAATGATCTCGATTTGAAGA | 16 | |

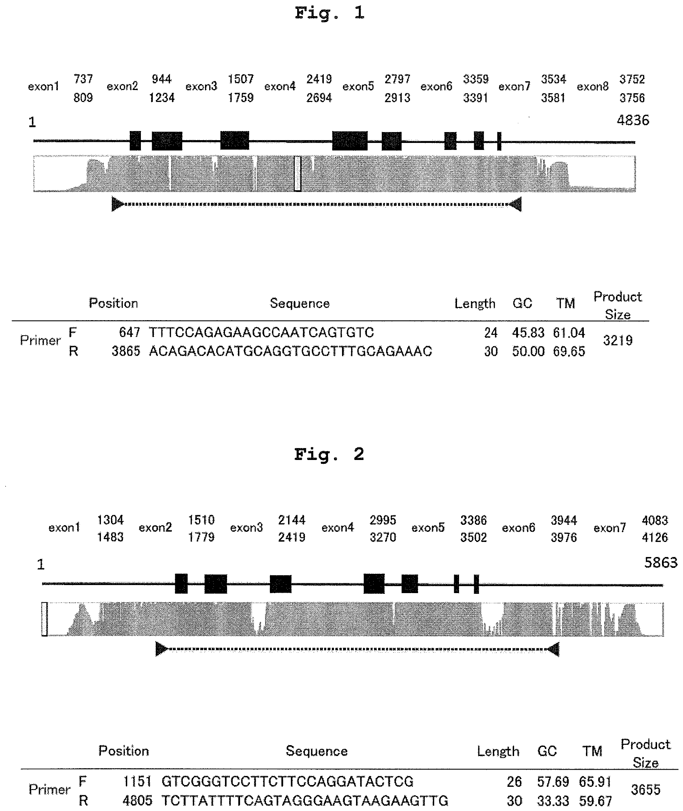

A schematic diagram showing the assembly results of large-scale sequencing in the HLA-A gene in the present invention and the positions of primers for amplification on the gene and an outline of the primers are shown in Fig. 1. In Table 1, the primer set containing a primer consisting of the base sequence shown in SEQ ID NO: 1 and a primer consisting of the base sequence shown in SEQ ID NO: 2 can specifically amplify HLA-A gene (this primer set is to be abbreviated as "HLA-A gene amplification primer set"). The HLA-A gene amplification primer set is designed to amplify the region of from the upstream side of exon 1 to the downstream side of exon 8 of HLA-A gene in the human genome base sequence (reference sequence: hg19).

A schematic diagram showing the assembly results of large-scale sequencing in the HLA-B gene in the present invention and the positions of primers for amplification on the gene and an outline of the primers are shown in Fig. 2. In Table 1, the primer set containing a primer consisting of the base sequence shown in SEQ ID NO: 3 and a primer consisting of the base sequence shown in SEQ ID NO: 4 can specifically amplify HLA-B gene (this primer set is to be abbreviated as "HLA-B gene amplification primer set"). The HLA-B gene amplification primer set is designed to amplify the region of from the upstream side of exon 1 to the downstream side of exon 7 of HLA-B gene in the human genome base sequence (reference sequence: hg19).

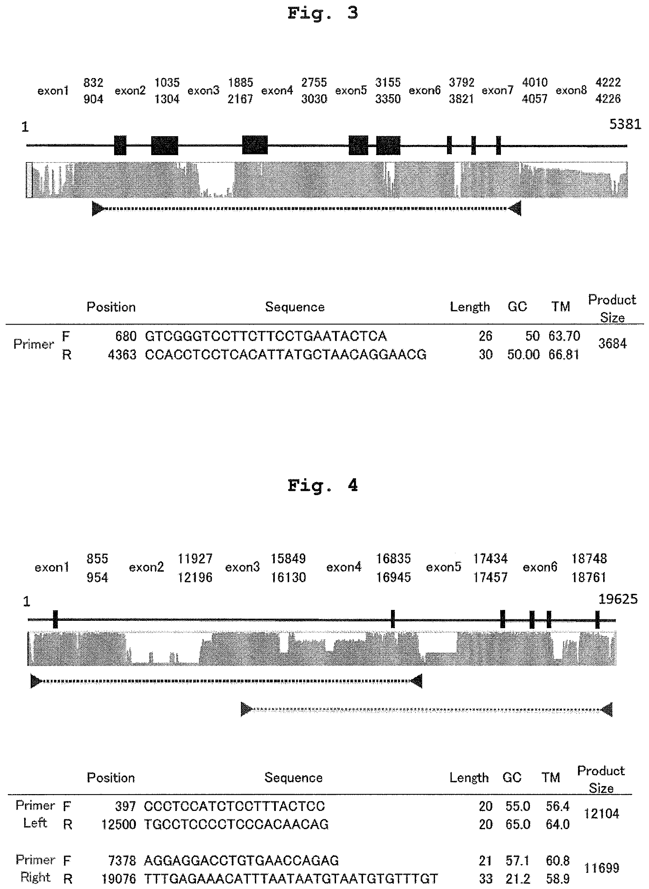

A schematic diagram showing the assembly results of large-scale sequencing in the HLA-C gene in the present invention and the positions of primers for amplification on the gene and an outline of the primers are shown in Fig. 3. In Table 1, the primer set containing a primer consisting of the base sequence shown in SEQ ID NO: 5 and a primer consisting of the base sequence shown in SEQ ID NO: 6 specifically amplifies HLA-C gene (this primer set is to be abbreviated as "HLA-C gene amplification primer set"). The HLA-C gene amplification primer set is designed to amplify the region of from the upstream side of exon 1 to the downstream side of exon 8 of HLA-C gene in the human genome base sequence (reference sequence: hg19).

A schematic diagram showing the assembly results of large-scale sequencing in the HLA-DRB1 gene in the present invention and the positions of primers for amplification on the gene and an outline of the primers are shown in Fig. 4. In Table 1, the primer set containing a primer consisting of the base sequence shown in SEQ ID NO: 7 and a primer consisting of the base sequence shown in SEQ ID NO: 8 specifically amplifies a part of HLA-DRB1 gene (this primer set is to be abbreviated as "HLA-DRB1 gene amplification primer set 1"). The HLA-DRB1 gene amplification primer set 1 is designed to amplify the region of from the upstream side of exon 1 to the downstream side of exon 2 of HLA-DRB1 gene in the human genome base sequence (reference sequence: hg19). In addition, the primer set containing a primer consisting of the base sequence shown in SEQ ID NO: 9 and a primer consisting of the base sequence shown in SEQ ID NO: 10 specifically amplifies a part of HLA-DRB1 gene (this primer set is to be abbreviated as "HLA-DRB1 gene amplification primer set 2"). The HLA-DRB1 gene amplification primer set 2 is designed to amplify the region of from the intron region between exon 1 and exon 2 to the downstream side of exon 6 of HLA-DRB1 gene in the human genome base sequence (reference sequence: hg19). Since the HLA-DRB1 gene has a long gene length, it is preferable to perform PCR using the aforementioned two kinds of primer sets to give robustness against degradation of DNA.

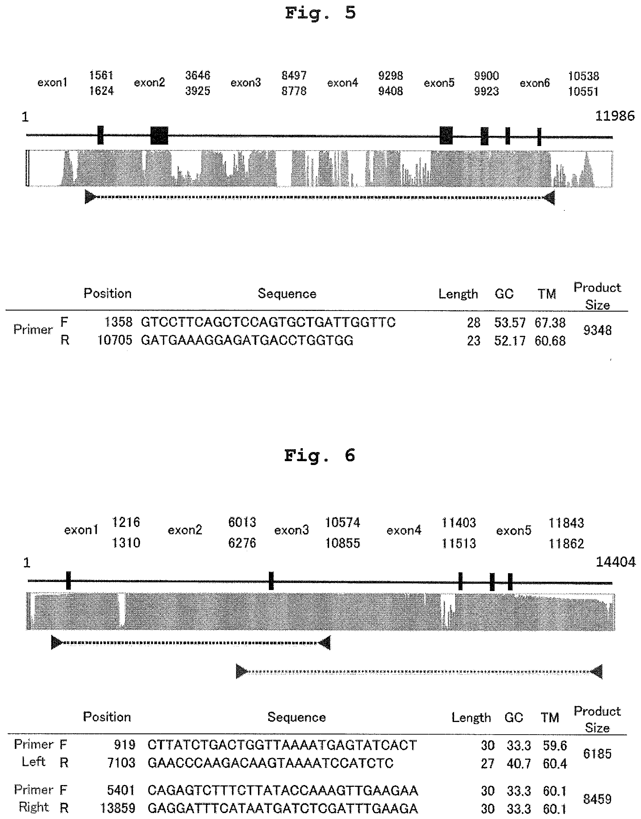

A schematic diagram showing the assembly results of large-scale sequencing in the HLA-DQB1 gene in the present invention and the positions of primers for amplification on the gene and an outline of the primers are shown in Fig. 5. In Table 1, the primer set containing a primer consisting of the base sequence shown in SEQ ID NO: 11 and a primer consisting of the base sequence shown in SEQ ID NO: 12 specifically amplifies HLA-DQB1 gene (this primer set is to be abbreviated as "HLA-DQB1 gene amplification primer set"). The HLA-DQB1 gene amplification primer set is designed to amplify the region of from the upstream side of exon 1 to the downstream side of exon 6 of HLA-DQB1 gene in the human genome base sequence (reference sequence: hg19).

A schematic diagram showing the assembly results of large-scale sequencing in the HLA-DPB1 gene in the present invention and the positions of primers for amplification on the gene and an outline of the primers are shown in Fig. 6. In Table 1, the primer set containing a primer consisting of the base sequence shown in SEQ ID NO: 13 and a primer consisting of the base sequence shown in SEQ ID NO: 14 specifically amplifies a part of HLA-DPB1 gene (this primer set is to be abbreviated as "HLA-DPB1 gene amplification primer set 1"). The HLA-DPB1 gene amplification primer set 1 is designed to amplify the region of from the upstream side of exon 1 to the downstream side of exon 2 of HLA-DPB1 gene in the human genome base sequence (reference sequence: hg19). In addition, the primer set containing a primer consisting of the base sequence shown in SEQ ID NO: 15 and a primer consisting of the base sequence shown in SEQ ID NO: 16 specifically amplifies a part of HLA-DPB1 gene (this primer set is to be abbreviated as "HLA-DPB1 gene amplification primer set 2"). The HLA-DPB1 gene amplification primer set 2 is designed to amplify the region of from the upstream side of exon 2 to the downstream side of exon 5 of HLA-DPB1 gene in the human genome base sequence (reference sequence: hg19). Since the HLA-DPB1 gene has a long gene length, it is preferable to perform PCR using the aforementioned two kinds of primer sets to give robustness against degradation of DNA.

The primer constituting the primer set of the present invention (hereinafter to be abbreviated as "the primer of the present invention") includes not only a nucleic acid consisting of the base sequence shown in each of the aforementioned SEQ ID NOs, but also a variant thereof having a base sequence that hybridizes to a base sequence complementary to the base sequence shown in each SEQ ID NO under stringent conditions and similarly having a function as a primer.

The "variant thereof having a base sequence that hybridizes to a base sequence complementary to the base sequence shown in each SEQ ID NO under stringent conditions and similarly having a function as a primer" is an oligonucleotide that hybridizes to an oligonucleotide consisting of a base sequence complementary to the base sequence shown in each SEQ ID NO under stringent conditions. For example, a "variant thereof having a base sequence that hybridizes to a base sequence complementary to the base sequence shown in SEQ ID NO:1 under stringent conditions and similarly having a function as a primer" has a function as a primer similar to that of an oligonucleotide consisting of the base sequence shown in SEQ ID NO:1, namely, a function capable of PCR amplification of a part of the HLA-A gene by combining with a primer consisting of the base sequence shown in SEQ ID NO: 2. Similarly, a "variant thereof having a base sequence that hybridizes to a base sequence complementary to the base sequence shown in SEQ ID NO:2 under stringent conditions and similarly having a function as a primer" is an oligonucleotide that hybridizes to an oligonucleotide consisting of a base sequence complementary to the base sequence shown in SEQ ID NO:2 under stringent conditions and has a function as a primer similar to that of an oligonucleotide consisting of the base sequence shown in SEQ ID NO:2, namely, a function capable of PCR amplification of a part of the HLA-A gene by combining with a primer consisting of the base sequence shown in SEQ ID NO: 1.

In such a variant, one or more nucleotides are substituted, deleted, inserted or added in the original oligonucleotide as long as the function as a primer is maintained.

The stringency during hybridization is known to be a function of temperature, salt concentration, primer strand length, GC content of the nucleotide sequence of the primer and the concentration of the chaotropic agent in the hybridization buffer. As the stringent conditions, the conditions described in Sambrook, J. et al. (1998) Molecular Cloning: A Laboratory Manual (2nd ed.), Cold Spring Harbor Laboratory Press, New York and the like can be used. The stringent temperature condition is not less than about 30°C, more preferably not less than about 37°C, most preferably not less than about 42°C. Other conditions include hybridization time, concentration of washing agents (e.g., SDS), presence or absence of carrier DNA and the like, and various stringencies can be determined by combining these conditions.

The primer of the present invention consists of a nucleic acid, and the nucleic acid is preferably a single-stranded nucleic acid. In the present invention, the nucleic acid means a molecule in which a nucleotide and a molecule having an equivalent function to that of the nucleotide are polymerized. For example, DNA, RNA and a polymer of RNA and DNA can be mentioned. When RNA is contained, "T(thymine)" in the DNA sequence is referred to as "U(uracil)" in the base sequence. The primer of the present invention can be produced by any chemical synthesis method well known to those of ordinary skill in the art. For example, it can be produced using an enzyme such as nuclease and the like, or can also be produced using a commercially available DNA/RNA automatic synthesizer (Applied Biosystems, Beckman Instruments etc.). In these primers, the constituting nucleic acid may be further modified freely. For example, in the primer of the present invention, the 5'-terminal or 3'-terminal may contain a labeling substance (e.g., fluorescent molecule, dye molecule, radioisotope, organic compound such as digoxigenin or biotin, and the like) and/or an addition sequence (loop primer part used in LAMP method and the like) to facilitate detection or amplification of the primer. The primer of the present invention may be phosphorylated or aminated at the 5-'terminal. The primer of the present invention may contain only a natural base or a modified base. Examples of the modified base include, but are not limited to, deoxyinosine, deoxyuracil, phosphorothioated base and the like. Furthermore, the primer of the present invention may contain any oligonucleotide derivative containing a phosphorothioate bond, a phophoroamidate bond and the like, or may contain peptide-nucleic acid (PNA) containing a peptide nucleic acid bond.

The primer of the present invention is preferably isolated or purified. Being "isolated or purified" means that an operation to eliminate components other than the object component from a natural or synthesized state is applied. The purity of the isolated or purified primer (percentage of target primer contained in total nucleic acid) in (w/w)% is generally not less than 50%, preferably not less than 70%, more preferably not less than 90%, most preferably not less than 95% (e.g., 100%). The purity of the primer may be appropriately changed according to the solvent and the state of solid or liquid. The unit of the purity may be (w/v)% or (v/v)%, and the desirable purity can be calculated as appropriate, taking into consideration the above-mentioned definition of purity in (w/w)%.

These primers can be provided as a solid in a dry state or in the state of alcohol precipitation, or can also be provided by being dissolved in water or a suitable buffer (e.g., TE buffer and the like).

A plurality of HLA genes can be amplified together by combining a plurality of the primer sets of the present invention. Therefore, the present invention provides primer sets including not less than two primer sets selected from the primer sets of the present invention recited in Table 1. Furthermore, using all primers recited in Table 1, multiplex PCR capable of amplifying a plurality of the above-mentioned important 6 kinds of HLA genes (i.e., HLA-A, HLA-B, HLA-C, HLA-DRB1, HLA-DQB1 and HLA-DPB1) can be performed, whereby highly efficient, highly uniform and economical typing of HLA gene can be realized. Since HLA-DRB1 gene amplification primer set 1 and HLA-DRB1 gene amplification primer set 2 have overlapping regions, multiplex PCR is preferably performed separately. Similarly, multiplex PCR is preferably performed separately for HLA-DPB1 gene amplification primer set 1 and HLA-DPB1 gene amplification primer set 2 containing primers shown in SEQ ID NO: 15 or 16. Therefore, in a preferred embodiment, the present invention provides a primer set containing all the primers of the present invention recited in Table 1.

When multiplex PCR is performed using the primer set of the present invention, PCR is performed by placing plural primer sets for amplifying the object HLA gene in the same tube (container). Since HLA-DRB1 gene amplification primer set 1 and HLA-DRB1 gene amplification primer set 2 have overlapping regions, they are preferably placed in separate containers. Similarly, HLA-DPB1 gene amplification primer set 1 and HLA-DPB1 gene amplification primer set 2 are also preferably placed in separate containers. In a preferred embodiment, the present invention provides a method for performing PCR of 2 containers together in which one container contains a mixture of HLA-A gene amplification primer set, HLA-C gene amplification primer set, HLA-DRB1 gene amplification primer set 1 and HLA-DPB1 gene amplification primer set 1, and the other container contains a mixture of HLA-B gene amplification primer set, HLA-DRB1 gene amplification primer set 2, HLA-DQB1 gene amplification primer set, and HLA-DPB1 gene amplification primer set 2.

In the present invention, DNA to be a template for PCR can be prepared from a test sample. The test sample is not particularly limited and includes, for example, body fluid samples such as blood, urine and the like, cells such as mouth cavity mucosa and the like, and body hair such as hair and the like.

DNA can be prepared using known methods such as a proteinase K/phenol extraction method, a phenol/chloroform extraction method, an alkali dissolution method, a boiling method and the like. It is possible to prepare a highly pure DNA rapidly and conveniently from a trace sample by using a commercially available DNA/RNA extraction kit.

The reaction conditions of PCR for HLA by using the primer sets of the present invention may be, for example, the following conditions.

heat denaturation step (e.g., 92 - 98°C)
annealing step (e.g., 55 - 72°C)
elongation step (e.g., 65 - 80°C)

In the above-mentioned PCR, the annealing step and the elongation step may be performed in one step (shuttle method), or a method including setting the annealing temperature higher and gradually lowering the temperature in each cycle may be adopted (touchdown method). Alternatively, they may be combined. When the shuttle method is performed, the temperature of the annealing step and the elongation step is typically 65 - 72°C.

Then, the base sequence of the obtained amplification product can be determined by a general method. It is preferable to determine the base sequence by using a next-generation sequencer (NGS). As for the next-generation sequencing, for example, Experiment Medicine (SUPPLEMENTAL) "Standard Protocols on Next Generatin Sequencing", 2014 (YODOSHA CO., LTD.) and the like can be referred to. Examples of NGS used for such purpose include, but are not limited to, apparatuses manufactured by Illumina (e.g., MiSeq, HiSeq2500), apparatuses manufactured by Life Technologies (e.g., Ion Proton, Ion PGM), apparatuses manufactured by Roche Diagnostic (e.g., GS FLX+, GS Junior) and the like.

A method for sequencing using NGS varies according to the kind of NGS and can be performed, for example, according to each company's manual (e.g., Nextera$^R$ XT DNA Library Prep Reference Guide). Paired-end analysis is preferably used for sequencing the obtained sample. In the following, a summary of procedure of sequencing using MiSeq of Illumina, Inc. is described. Even when other apparatus (Ion Proton manufactured by Life Technologies, GS FLX+ of Roche Diagnostics K.K. and the like) is used, a base sequence can be similarly determined by a method suitable for the apparatus.

1. The amplification product of each sample is subjected to Tagmentation using a kit (e.g., Nextera XT DNA Sample Preparation Kit (Illumina, Inc.)).
2. Using a kit (e.g., Nextera XT v2 Index Kit Set A, B, C, D (Illumina, Inc.)), Index sequences, which are different for samples, are added to the both sides of the obtained fragment sequences and PCR is performed.
3. Amplification product is purified.
4. Using the amplification product from each sample, library size is confirmed.
5. The concentration is adjusted between samples.
6. The amplification products of the samples are pooled, and quantitative PCR is performed for the pooled amplification products.
7. The pooled amplification products are sequenced using MiSeq.

In this way, the genotype of the HLA gene can be determined (typing) using a database based on the base sequence information (hereinafter read) of the amplification product obtained by NGS. Examples of the database include IPD-IMGT/HLA database in which more than 17,000 kinds of alleles are registered, HLA dictionary newly created by the present inventors, and the like. The HLA dictionary is created by extracting the entire base sequences of exons and introns of all HLA alleles registered in IPD-IMGT/HLA database, grouping them by base sequence patterns, and recording these patterns and information of alleles belonging thereto. Examples of the typing method based on the base sequence information of the amplification product obtained by NGS include, but are not limited to, a method including mapping the read to alleles of multiple HLA genes, applying the results thereof to a linear programming problem and detecting a best fit allele pair (OptiType) (Szolek, A. et al., (2014) OptiType: precision HLA typing from next-generation sequencing data. Bioinformatics, 30, 3310-3316.), a method including de novo assembling the read mapping results and determining alleles from the results, (HLA reporter) (Huang, Y. et al., (2015) HLA reporter: a tool for HLA typing from next generation sequencing data. Genome Med, 7, 25.).), comparing allele pairs from exon of antigen-presenting site and sequentially expanding the searching range to all exons, thus enabling typing of all alleles of IPD-IMGT/HLA (HLA-HD) (Kawaguchi, et al., (2017) HLA-HD: An accurate HLA typing algorithm for next-generation sequencing data. Human Mutation, 38, 788-797) and the like.

Alternatively, HLA typing may be performed without using NGS. Examples of such method include, but are not limited to, analysis methods using amplification products by long PCR such as array-based PCR-SSOP method, Sanger method-based PCR-SBT method, PCR-PHFA method, PCR-SSCP method and the like.

The present invention also provides a kit usable in PCR using the primer set of the present invention. The kit of the present invention may contain other reagents necessary for PCR in addition to the above-mentioned primer set of the present invention. When the aforementioned reagent does not adversely affect the reaction after preservation in coexistence with the primer set of the present invention, it can be mixed with the primer set and contained in the kit. Alternatively, the aforementioned reagent and the primer set of the present invention may be provided separately without being mixed. Examples of the aforementioned reagent include DNA extraction reagent, DNA polymerase enzyme, dNTP, reaction buffer, DNA molecule containing target sequence to be the positive control in PCR, instructions and the like. The aforementioned DNA polymerase enzyme may be a commercially available product. Examples of the DNA polymerase enzyme include, but are not limited to, PrimeSTAR GXL DNA Polymerase, Tks Gflex DNA Polymerase, TaKaRa LA Taq manufactured by TaKaRa, and Long PCR Enzyme Mix manufactured by Thermo Scientific.

While the present invention is explained in further detail by referring to Examples, the present invention is not limited by the Examples.

[Examples]

Example 1 Design of multiplex PCR primer set of Classical HLA gene

The design procedure of primers for performing multiplex PCR of 6 kinds of Classical HLA genes (HLA-A, -B, -C, -DRB1, - DQB1, -DPB1) is described. Bowtie2 (ver. 4.1.2) was used for mapping all reads obtained by the next-generation sequencer.

I. Assembly of full-length gene from sequencing results of HLA gene

1. Sequencing of HLA gene by conventional method

Using existing primer set [Hosomichi, K. et al. (2013) Phase-defined complete sequencing of the HLA genes by next-generation sequencing. BMC Genomics, 14:355.], multiplex PCR with 2 sets of each 3 genes was performed for 768 samples (unreleased samples) (see Table 2). For DRB1, primer with modified sequence was added. For experiment conditions, refer to Table 3. The obtained amplification product was sequenced by MiSeq.

[Table 2]

Table 2. Primer set of Hosomichi

| primer | HLA | forward (F)/reverse (R) | set | primer sequence | final concentration (μM) | SEQ ID NO: |
|---|---|---|---|---|---|---|
| HLA-A_F7 | A | F | 1 | ATCCTGGATACTCACGACGCGGAC | 0.1 | 17 |
| HLA-A_R8 | | R | 1 | CATCAACCTCTCATGGCAAGAATTT | | 18 |
| HLA-B_F3 | B | F | 1 | AGGTGAATGGCTCTGAAAATTTGTCTC | 0.218 | 19 |
| HLA-B_R3 | | R | 1 | AGAGTTTAATTGTAATGCTGTTTTGACACA | | 20 |
| HLA-C_F4 | C | F | 2 | GGCCGCCTGTACTTTTCTCAGCAG | 0.05 | 21 |
| HLA-C_R3 | | R | 2 | CCATGGTGAGTTTCCCTGTACAAGAG | | 22 |
| HLA-DRB1_F1 | DRB1 | F | 2 | TGATTGACTTGCTGGCTGGTTTCTCATC | 0.15 | 23 |
| HLA-DRB1_R2 | | R | 2 | GCATCCACAGAATCACATTTTCTAGTGTT | | 24 |
| HLA-DRB1_R50* | | R | 2 | GCATCCACAGAATCACATTTTCCAGTATT | | 25 |
| HLA-DQB1_F6 | DQB1 | F | 2 | TCATGTGCTTCTCTTGAGCAGTCTGA | 0.1 | 26 |
| HLA-DQB1_R7 | | R | 2 | TGTGACAGCAATTTTCTCTCCCCT | | 27 |
| HLA-DPB1_F1 | DPB1 | F | 1 | TGGTCCAACAGGATCACATTTATAAGTGT | 0.3 | 28 |
| HLA-DPB1_R1 | | R | 1 | CCCAGTTTGGATGGTCTCTCAGCTCTT | | 29 |
| * added in the Center for Genomic Medicine, Kyoto University | | | | | | |

[Table 3]

| Table 3. Experiment conditions in multiplex PCR | | |
|---|---|---|
| temperature | time | cycle number |
| set 1 | | |
| 94°C | 2 min | 1 |
| 98°C | 10 sec | 5 |
| 74°C | 10 min | |
| 98°C | 10 sec | 5 |
| 72°C | 10 min | |
| 98°C | 10 sec | 5 |
| 70°C | 10 min | |
| 98°C | 10 sec | 15 |
| 62°C | 15 sec | |
| 68°C | 15 min | |
| 68°C | 5 min | 1 |
| 16°C | ∞ | 1 |
| set 2 | | |
| 94°C | 2 min | 1 |
| 98°C | 10 sec | 27 |
| 59.5°C | 15 sec | |
| 67.5°C | 10 min | |
| 68°C | 10 min | 1 |
| 16°C | ∞ | 1 |

2. mapping to HLA dictionary

The sequencing result of MiSeq was mapped using bowtie2 for the sequence information of exons and introns of all HLA alleles obtained from IPD-IMGT/HLA database. The weight of read in each gene was calculated based on the mapping result. The calculation method of the weight is briefly explained below.

The mapping results are read and the correspondence of reads that match each exon is checked. It is examined whether 50% or more of the reads overlap with the base sequence of any exon contained in the HLA dictionary and the both base sequences completely match in the overlapping range or whether 50% or more of the reads overlap with the base sequence of any exon contained in the HLA dictionary and the both base sequences match within 2 bases mismatch in the overlapping range. The matched read for each gene is weighted according to the number of alleles corresponding to the matched sequence in each exon and intron. The weight of the read for each gene is obtained by calculating the following formula 1

$$W_r^G = \frac{F_r^G}{\sum_{g \in \Lambda} F_r^{g'}}$$

$$K_g^r = \{X \in K_g \,|\, m_X(r) > 0\},$$

$$\text{if } K_g^r \neq \emptyset, \qquad F_r^g = \prod_{X \in K_g^r} \frac{m_X(r)}{N_X};$$

otherwise, $F_r^g = 0$
wherein

$r$: read
$G$: gene
$w_r^G$ : weight given to gene $G$ relating to read $r$
$\Lambda$: set of all HLA genes
$g$: gene (one of $\Lambda$)
$X$: exon or intron
$m_X(r)$: total number of alleles that matched read $r$ on $X$
$K_g^r$ : set of exons and introns in which $m_X(r)>0$ in gene $g$
$Kg$: set of all exons and introns in gene $g$
$N_X$: total number of alleles containing exon $X$

separately for the paired-end read and summarizing by the following formula 2

$$w_{r_{f,r}}^G = \min(w_{r_f}^G, w_{r_r}^G)$$

wherein

$r_{f,r}$: pair of paired-end reads
$r_f$: forward read
$r_r$: reverse read
$w_{r_f}^G$ : weight of forward read (according to formula 1)
$w_{r_r}^G$ : weight of reverse read (according to formula 1)
$w_{r_{f,r}}^G$ : weight given to gene G of paired-end read (common to forward read and reverse read.

The following procedure was performed separately for each HLA gene (gene G). The weight calculated above was added to the count of coverage.

3. Mapping to gene and detection of different position

The read group that obtained positive (>0) weight in gene G was mapped to all alleles (hereinafter complete allele) of gene G whose full length sequences including intron are registered in IPD-IMGT/HLA. The reads mapped within 5 bases mismatch to the complete allele were collected, and the coverage of each base at the mapped position was calculated. A sequence obtained by selecting a base showing the maximum calculated coverage at each position was created (hereinafter to be abbreviated as "consensus sequence"). When, at a certain position, the coverage of a base different from the consensus sequence is 10% or more of the coverage of the base corresponding to the consensus sequence, the position was taken as a different position and the base sequence thereof was taken as a different sequence. When multiple bases have a coverage of 10% or more, one with the largest coverage was selected. Two classes with the consensus and difference as IDs were prepared, they were compared to determine which of the consensus and different sequence is contained more in the reads mapped to locations containing different positions, and the reads were taken as belonging to the class contained more. A read that contained the same number or did not contain even a single different position was regarded as belonging to the both classes.

### 4. Inversion and elimination of different sequence

The coverage was recalculated only by the set of reads belonging to the class of consensus. When the coverage of the consensus sequence at a different position is lower than the coverage of the different sequence, the bases corresponding to the consensus sequence and different sequence were replaced at the different position. Thereafter, the coverage was calculated only by the set of reads belonging to the class on the difference side. When the coverage of the consensus sequence at a different position is higher than the coverage of the different sequence, the different position was eliminated. The reads of step 3 were classified again relative to the entire reads based on the inverted and eliminated information, inversion of the consensus sequence and different sequence at the different position and elimination of the different position were performed. This operation was repeated until inversion and elimination of different positions did not occur.

### 5. Selection of optimal allele pair

Steps 3, 4 were performed in entire complete alleles, and classified sets of reads were obtained. Any two complete alleles were taken as A, B, sets of reads belonging to the class of consensus of A, B were respectively taken as C(A), C(B), and sets of reads belonging to the class of difference of A, B were respectively taken as D(A), D(B). In each union of C(A)∪C(B), C(A)∪D(B), D(A)∪C(B), D(A)∪D(B), the total number of reads after applying the weight of formula 2 (equation 2) was calculated. The number of reads in all unions of all combinations of complete allele pairs was compared, and the allele pair and the kind of union showing the highest read number were selected. The pair search included a combination of A and B being the same complete allele.

### 6. Correction of different position

When the pair selected in step 5 contained different complete alleles, the correspondence between different positions of the two complete alleles is unknown. Thus, the different positions were matched using the relationship between the mapped reads. First, a set of mapped reads including this position was detected at each different position of the complete allele. The set of reads includes those belonging to the both classes of consensus and difference. The number of common reads for all combinations of sets of reads at different positions between two complete alleles was calculated, and the different positions corresponding to the set were made to correspond to the order of the combination of sets with many common reads as the same different positions between complete alleles. when a corresponding different position did not exist on one complete allele side, the different position was eliminated.

### 7. Reclassing and determination of consensus sequence

With respect to the allele pair determined in step 5 and the class corresponding thereto, and different positional relationship between them, each read was determined by the method of step 3 as to which complete allele side it belongs to. Consensus sequences were created from the set of reads of each complete allele obtained by the determination results.

### 8. Sequential update of consensus sequence

1) All reads were remapped to the two consensus sequences obtained in step 7. 2) The number of mismatch in two mapping results was compared and each read was made to belong to the one showing a smaller number. When the number of mismatch was the same, the read was made to belong to the both. Using the set of reads belonging to each, a consensus sequence was created again. A position where the coverage of the consensus did not reach 5 read number was "N". Steps 1) and 2) were performed again on 3) two sequences obtained. This was repeated until there was no update in both sequences.

### 9. Homo-hetero determination

The set of reads in two consensus sequences finally obtained in step 8 was compared. When, at this time, the number of reads belonging to only one allele is more than 10 times the number of reads belonging only to the other, the smaller allele was eliminated and the remaining allele was output as a homo allele by the consensus sequence. Otherwise, the consensus sequence of both alleles was output. The output sequence is taken as an assembly sequence.

### II. Design of novel primer using assembly sequence

Previously, in each of 768 DNA samples stored in the Center for Genomic Medicine affiliated with Graduate School of Medicine of Kyoto University (hereinafter to be abbreviated as "Center for Genomic Medicine"), an assembly sequence of 6 kinds of HLA genes was created using method of I.

1. Elimination of short assembly results

Sequences in which the number of base sequences did not reach the following lengths were removed from the obtained assembly sequences. On this occasion, the "N" part was subtracted from the number of sequences

    HLA-A: 2,800bp
    HLA-B: 2,800bp
    HLA-C: 2,800bp
    HLA-DRB1: 10,000bp
    HLA-DQB1: 6,500bp
    HLA-DPB1: 9,500bp

When "N" was contained in 5% or more of the entire sequence, the assembly sequence was removed regardless of the length.

2. Execution of multiple alignment

Multiple alignment was performed using MUSCLE (ver 3.8.31) [Edgar, R.C. (2004) MUSCLE: multiple sequence alignment with high accuracy and high throughput. Nucleic Acids Res., 32:1792-1797.] on the set of assembly sequences of each gene remaining in step 1.

3. Elimination of rare insertion

When an insertion of 20 or more bases occurred (section with coverage of 1 continues over 20 bases) by one assembly sequence in a certain section of the consensus sequence obtained from the alignment results, the section was eliminated and narrowed the distance between the alignment positions.

4. Masking of difference section, Exon section

The position where coverage of the consensus sequence obtained in Step 3 was not more than 95% of the number of assembly sequences was masked. In addition, the position corresponding to the exon on the consensus sequence was searched from the sequence data of known HLA genes by BLAST, a software for searching similar sequences, and the matched exon section was masked.

5. Selection of primer candidate

Using Primer3 [Untergasser, A. et al. (2012) Primer3--new capabilities and interfaces. Nucleic Acids Res., 40:e115.], the primer pair candidate was determined. The primer satisfied the following standards.
Not containing the region masked in Step 4.
A, B, C, DQB1 include all translation regions in one pair.
Two pairs were prepared for DRB1 and DPB1, the both pairs contain exon 2 and include the translation region by two forward and backward pairs.
The primer pair of each gene is specific to any other HLA gene. Specific position is present as inner as possible (3'-side position for 5'-side primer and 5'-side position for 3'-side primer).
Options of Primer3 are as follows.

    PRIMER_OPT_SIZE=30
    PRIMER_MIN_SIZE=20
    PRIMER_MAX_SIZE=35
    PRIMER_MIN_TM=40
    PRIMER_MAX_TM=90
    PRIMER_MIN_GC=10
    PRIMER_MAX_GC=90
    PRIMER_MAX_NS_ACCEPTED=1
    P3_FILE_FLAG=1

PRIMER_MAX_SELF_END=3
PRIMER_PAIR_MAX_COMPL_END=3
PRIMER_MAX_HAIRPIN_TH=60

When plural candidates satisfied the above, an optimal one was selected in consideration of the sequence specificity and the score of Primer3. This operation was performed artificially. Information of the obtained primer sets is shown in Table 4.

[Table 4]

Table 4. HLA primer by design of Kyoto University Center for Genomic Medicine

| primer | HLA | forward (F)/reverse (R) | tube | primer sequence (5'→3') | SEQ ID NO: | predicted length (Kb) | final concentration (uM) |
|---|---|---|---|---|---|---|---|
| Kyoto_HLA_A_F | A | F | 1 | TTTCCAGAGAAGCCAATCAGTGTC | 1 | 3.1 | 0.06 |
| Kyoto_HLA_A_R | | R | 1 | ACAGACACATGCAGGTGCCTTTGCAGAAAC | 2 | | |
| Kyoto_HLA_B_F | B | F | 2 | GTCGGGTCCTTCTTCCAGGATACTCG | 3 | 3.1 | 0.25 |
| Kyoto_HLA_B_R | | R | 2 | TCTTATTTTCAGTAGGGAAGTAAGAAGTTG | 4 | | |
| Kyoto_HLA_C_F | C | F | 1 | GTCGGGTCCTTCTTCCTGAATACTCA | 5 | 3.2 | 0.03 |
| Kyoto_HLA_C_R | | R | 1 | CCACCTCCTCACATTATGCTAACAGGAACG | 6 | | |
| Kyoto_HLA_DRB1_FF | DRB1 | F | 1 | CCCTCCCATCTCCTTTACTCC | 7 | 6.3 - 11.3 | 1.00 |
| Kyoto_HLA_DRB1_FR | | R | 1 | TGCCTCCCCTCCCACAACAG | 8 | | |
| Kyoto_HLA_DRB1_RF | | F | 2 | AGGAGGACCTGTGAACCAGAG | 9 | 7.5 - 10.7 | 1.00 |
| Kyoto_HLA_DRB1_RR | | R | 2 | TTTGAGAAACATTTAATAATGTAATGTGTTTGT | 10 | | |
| Kyoto_HLA_DQB1_F | DQB1 | F | 2 | GTCCTTCAGCTCCAGTGCTGATTGGTTC | 11 | 6.7 | 0.09 |
| Kyoto_HLA_DQB1_R | | R | 2 | GATGAAAGGAGATGACCTGGTGG | 12 | | |

| primer | HLA | forward (F)/ reverse (R) | tube | primer sequence (5'→3') | SEQ ID NO: | predicted length (Kb) | final concentration (uM) |
|---|---|---|---|---|---|---|---|
| Kyoto_HLA DPB1_FF | DPB1 | F | 1 | CTTATCTGACTGGTTAAAATGAGTATCACT | 13 | 6.0 | 0.18 |
| Kyoto_HLA_ DPB1_FR | | R | 1 | GAACCCAAGACAAGTAAAATCCATCTC | 14 | | |
| Kyoto_HLA DPB1_RF | | F | 2 | CAGAGTCTTTCTTATACCAAAGTTGAAGAA | 15 | 8.2 | 0.90 |
| Kyoto_HLA_ DPB1_RR | | R | 2 | GAGGATTTCATAATGATCTCGATTTGAAGA | 16 | | |

17

Example 2 Multiplex PCR and NGS sequencing

I. long PCR

1. Multiplex long PCR targeting 6 genes of HLA-A, -B, -C, -DRB1, -DQB1, -DPB1 was performed on each of 384 DNA samples in total. Amplification was performed in the respective two sets below.

set 1: HLA-A, -C, -DPB1(FF,FR), -DRB1(FF,FR)
set 2: HLA-B, -DQB1, -DPB1(RF,RR), -DRB1(RF,RR)

Primer sequences and final concentrations are as shown in Table 3. The following were prepared for PCR.

DNA 25 ng
0.25 $\mu$L of Tks Gflex™ DNA Polymerase
6.25 $\mu$L 2 × Gflex™ PCR Buffer

After preparation to a final volume of 12.5 pL, Cool Start™ method (TaKaRa Inc.) was applied, and PCR reaction was performed under temperature condition and cycle number shown in Table 5. As PCR apparatus, GeneAmp[R] PCR System 9700 (Thermo Fisher Scientific inc., Waltham, MA, USA) was used.
2. Amplification product was purified by Agencourt[R] AMPure[R] XP Beads (Beckman Coulter, Brea, CA, USA).
3. Each sample (1 $\mu$L) was electrophoresed on agarose gel, and the presence or absence of the object band was confirmed. The assumed band size is as indicated in Table 3.
4. Equal moles of the two sets were mixed and each sample was prepared to have the final concentration of 0.2 ng/$\mu$L (2.5 $\mu$L) of the amplification product.

[Table 5]

Table 5. Experiment conditions for multiplex PCR

| temperature | time | cycle number |
| --- | --- | --- |
| 94°C | 2 min | 1 |
| 98°C | 10 sec | 20 |
| 72 - 0.3× (cycle -1)°C | 6 min - 3 × (cycle - 1) sec | |
| 98°C | 10 sec | 6 |
| 65°C | 5 min | |
| 65°C | 7 min | 1 |
| 4°C | ∞ | 1 |

II. Sequencing using next generation sequencer (MiSeq)

Protocols not specifically described below were in accordance with the manual of Illumina, Inc. (Nextera XT DNA Library Prep Reference Guide (15031942 Rev. D)).

1. The amplification product (0.5 ng) of each sample was subjected to Tagmentation by applying Nextera XT DNA Sample Preparation Kit (Illumina) (300 bp×2 paired-end read).
2. Using Nextera XT v2 Index Kit Set A, B, C, D (2.5 $\mu$L for each row, column), different Index sequence was added to the both sides of each of the obtained fragment sequences of 384 samples, and PCR was performed.

3. The amplification product was purified by Agencourt[R] AMPure[R] XP Beads.

4. Using amplification product (1 µL) from each sample, the library size was confirmed by Agilent 2100 Bioanalyzer and High Sensitivity DNA chip (Agilent Technology).

5. The concentration between samples was adjusted with Library Normalization Beads in the kit.

6. Equal amounts of the amplification products of 384 samples were pooled.

7. Quantitative PCR using KAPA Library Quantification Kit was performed on pooled amplification products (see Technical Data Sheet of KAPA Library Quantification Kit. Illumina[R] platforms for the detail).

8. Using MiSeq (Illumina), 6 HLA genes were sequenced in 384 samples.

9. It was confirmed whether about 15 GB data size (25 M paired-end read) was output.

III. 6 HLA genes sequencing preliminary experiment results using the primer set of the present invention

A preliminary experiment was performed using 384 samples stored in the Center for Genomic Medicine, designed primer sets for HLA gene and the above-mentioned experiment method. For comparison, 96 samples were simultaneously sequenced using the primer sets and experiment method in a previous report [Hosomichi, K. et al. (2013) Phase-defined complete sequencing of the HLA genes by next-generation sequencing. BMC Genomics, 14:355.]. Note that the comparison sample does not overlap with the preliminary experiment. The sequencing results of these samples by MiSeq were mapped to all HLA alleles with known full-length sequences in the IMGT/HLA database. The reads that matched any allele were collected and the average coverage for each gene was calculated. The gene length to be the denominator of the average coverage differs depending on the HLA allele. Thus, the average length of each gene in the allele set was used.

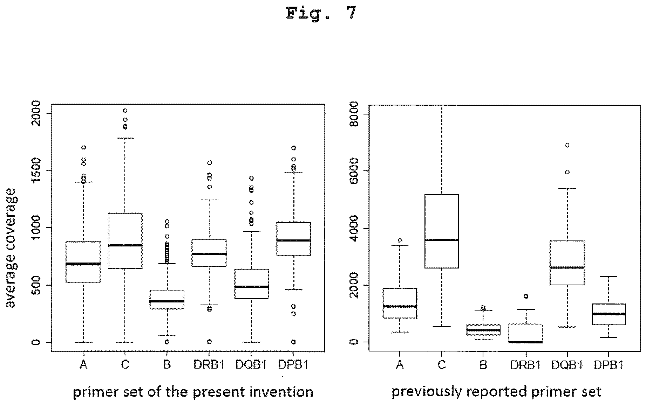

Fig. 7 shows the comparison results of the distribution of the average coverage of six kinds of genes between two kinds of primer sets. Due to the difference in the sample number used, the average coverage is higher in the existing set side (due to same output data size of MiSeq). In the results of primer set in a previous report, the average coverage of HLA-B and HLA-DRB1 was lower than with other genes. Particularly, the median value was 0 for HLA-DRB1, which means that an amplification product was not obtained in not less than half the samples. In contrast, it was confirmed that the primer set of the present invention shows low variation among genes, and the sequence of HLA-DRB1 can be read without any problem. Therefore, using the primer set of the present invention, the number of samples for one operation of the next-generation sequencer can be increased. Thus, the primer set of the present invention has a superior effect compared to the conventional primer sets.

This application is based on a patent application No. 2017-024397 filed in Japan (filing date: February 13, 2017), the contents of which are incorporated in full herein.

[Industrial Applicability]

The primer set of the present invention enables uniform PCR amplification, and can increase the number of samples to be used for one operation of a next-generation sequencer. Using the common sequence of large-scale samples of Japanese people obtained at the Center for Genomic Medicine, the frequency of occurrence of unidentified allele that does not match the primer is considered to be extremely low for Japanese people. Thus, it is suitable for automation of HLA typing since the reperformance rate is reduced. It is applicable to organ transplantation, highly safe compatible tests in the treatment using iPS cells in the future, and securing many donors. In addition, since the number of primers can be reduced, is easily arranged and is easily commercialized.

**Claims**

1. A primer set for HLA-A gene amplification comprising a primer consisting of the base sequence shown in SEQ ID NO: 1 and a primer consisting of the base sequence shown in SEQ ID NO: 2.

2. A primer set for HLA-B gene amplification comprising a primer consisting of the base sequence shown in SEQ ID NO: 3 and a primer consisting of the base sequence shown in SEQ ID NO: 4.

3. A primer set for HLA-C gene amplification comprising a primer consisting of the base sequence shown in SEQ ID NO: 5 and a primer consisting of the base sequence shown in SEQ ID NO: 6.

4. A primer set for HLA-DRB1 gene amplification comprising a primer consisting of the base sequence shown in SEQ ID NO: 7, a primer consisting of the base sequence shown in SEQ ID NO: 8, a primer consisting of the base sequence shown in SEQ ID NO: 9, and a primer consisting of the base sequence shown in SEQ ID NO: 10.

5. A primer set for HLA-DQB1 gene amplification comprising a primer consisting of the base sequence shown in SEQ ID NO: 11 and a primer consisting of the base sequence shown in SEQ ID NO: 12.

6. A primer set for HLA-DPB1 gene amplification comprising a primer consisting of the base sequence shown in SEQ ID NO: 13, a primer consisting of the base sequence shown in SEQ ID NO: 14, a primer consisting of the base sequence shown in SEQ ID NO: 15, and a primer consisting of the base sequence shown in SEQ ID NO: 16.

7. A primer set for HLA gene amplification comprising not less than two selected from the group consisting of the primer set according to claim 1, the primer set according to claim 2, the primer set according to claim 3, the primer set according to claim 4, the primer set according to claim 5, and the primer set according to claim 6.

8. A primer set for HLA gene amplification comprising all primer sets according to claims 1 to 6.

9. A method of sequencing an HLA gene comprising using the primer set according to any one of claims 1 to 8.

10. A method of typing an HLA gene comprising using base sequence information obtained by the method according to claim 9.

11. A kit for HLA gene amplification comprising the primer set according to any one of claims 1 to 8.

## Fig. 1

exon1 737 exon2 944 exon3 1507 exon4 2419 exon5 2797 exon6 3359 exon7 3534 exon8 3752
809 1234 1759 2694 2913 3391 3581 3756

1 4836

| | | Position | Sequence | Length | GC | TM | Product Size |
|---|---|---|---|---|---|---|---|
| Primer | F | 647 | TTTCCAGAGAAGCCAATCAGTGTC | 24 | 45.83 | 61.04 | 3219 |
| | R | 3865 | ACAGACACATGCAGGTGCCTTTGCAGAAAC | 30 | 50.00 | 69.65 | |

## Fig. 2

exon1 1304 exon2 1510 exon3 2144 exon4 2995 exon5 3386 exon6 3944 exon7 4083
1483 1779 2419 3270 3502 3976 4126

1 5863

| | | Position | Sequence | Length | GC | TM | Product Size |
|---|---|---|---|---|---|---|---|
| Primer | F | 1151 | GTCGGGTCCTTCTTCCAGGATACTCG | 26 | 57.69 | 65.91 | 3655 |
| | R | 4805 | TCTTATTTTCAGTAGGGAAGTAAGAAGTTG | 30 | 33.33 | 59.67 | |

## Fig. 3

| | | Position | Sequence | Length | GC | TM | Product Size |
|---|---|---|---|---|---|---|---|
| Primer | F | 680 | GTCGGGTCCTTCTTCCTGAATACTCA | 26 | 50 | 63.70 | 3684 |
| | R | 4363 | CCACCTCCTCACATTATGCTAACAGGAACG | 30 | 50.00 | 66.81 | |

## Fig. 4

| | | Position | Sequence | Length | GC | TM | Product Size |
|---|---|---|---|---|---|---|---|
| Primer Left | F | 397 | CCCTCCATCTCCTTTACTCC | 20 | 55.0 | 56.4 | 12104 |
| | R | 12500 | TGCCTCCCCTCCCACAACAG | 20 | 65.0 | 64.0 | |
| Primer Right | F | 7378 | AGGAGGACCTGTGAACCAGAG | 21 | 57.1 | 60.8 | 11699 |
| | R | 19076 | TTTGAGAAACATTTAATAATGTAATGTGTTTGT | 33 | 21.2 | 58.9 | |

## Fig. 5

| | | Position | Sequence | Length | GC | TM | Product Size |
|---|---|---|---|---|---|---|---|
| Primer | F | 1358 | GTCCTTCAGCTCCAGTGCTGATTGGTTC | 28 | 53.57 | 67.38 | 9348 |
| | R | 10705 | GATGAAAGGAGATGACCTGGTGG | 23 | 52.17 | 60.68 | |

## Fig. 6

| | | Position | Sequence | Length | GC | TM | Product Size |
|---|---|---|---|---|---|---|---|
| Primer Left | F | 919 | CTTATCTGACTGGTTAAAATGAGTATCACT | 30 | 33.3 | 59.6 | 6185 |
| | R | 7103 | GAACCCAAGACAAGTAAAATCCATCTC | 27 | 40.7 | 60.4 | |
| Primer Right | F | 5401 | CAGAGTCTTTCTTATACCAAAGTTGAAGAA | 30 | 33.3 | 60.1 | 8459 |
| | R | 13859 | GAGGATTTCATAATGATCTCGATTTGAAGA | 30 | 33.3 | 60.1 | |

Fig. 7

primer set of the present invention

previously reported primer set

## INTERNATIONAL SEARCH REPORT

| International application No. |
|---|
| PCT/JP2018/004703 |

**A. CLASSIFICATION OF SUBJECT MATTER**
Int.Cl. C12N15/09(2006.01)i, C12Q1/68(2018.01)i

According to International Patent Classification (IPC) or to both national classification and IPC

**B. FIELDS SEARCHED**

Minimum documentation searched (classification system followed by classification symbols)
Int.Cl. C12N15/09, C12Q1/68

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
CAplus/MEDLINE/EMBASE/BIOSIS(STN), JSTPlus/JMEDPlus/JST7580(JDreamIII), GenBank/EMBL/DDBJ/GeneSeq, PubMed

**C. DOCUMENTS CONSIDERED TO BE RELEVANT**

| Category* | Citation of document, with indication, where appropriate, of the relevant passages | Relevant to claim No. |
|---|---|---|
| A | WO 2014/065410 A1 (GENODIVE PHARMA INC.) 01 May 2014, claim 1, table 1 & JP 2016-010318 A | 1-11 |
| A | HOSOMICHI, K., et al., "Phase-defined complete sequencing of the HLA genes by next-generation sequencing.", BMC Genomics, 2013, vol. 14:355, pp. 1-16, page 14, left column, paragraph [0003], table S1 | 1-11 |
| A | JP 2011-500041 A (F. HOFFMANN-LA ROCHE AG.) 06 January 2011, claim 1, paragraphs [0070]-[0086] & WO 2009/049889 A1, claim 1, pages 19-35 & EP 2203567 A1 & US 2010/0086914 A1 & US 2010/0261189 A1 & US 2014/0141436 A1 | 1-11 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 February 2018 (27.02.2018) | 13 March 2018 (13.03.2018) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

# EP 3 581 652 A1

**INTERNATIONAL SEARCH REPORT**

| International application No. |
|---|
| PCT/JP2018/004703 |

C (Continuation).   DOCUMENTS CONSIDERED TO BE RELEVANT

| Category* | Citation of document, with indication, where appropriate, of the relevant passages | Relevant to claim No. |
|---|---|---|
| A | US 2012/0122719 A1 (HOGAN, M. E., et al.) 17 May 2012, claim 4, example 2 & US 2011/0117553 A1 & US 2016/0348171 A1 & WO 2011/059508 A2 & EP 2501827 A1 | 1-11 |
| A | US 2015/0005179 A1 (INSTANT CHEMISTRY INC.) 01 January 2015, claim 1, SEQ ID NO: 1-101 & WO 2014/205554 A1 | 1-11 |

Form PCT/ISA/210 (continuation of second sheet) (January 2015)

## REFERENCES CITED IN THE DESCRIPTION

### Patent documents cited in the description

- JP 2016010318 A

- JP 2017024397 A

### Non-patent literature cited in the description

- **HOSOMICHI, K. et al.** *BMC Genomics,* 2013, vol. 14, 355
- **EHRENBERG, P.K. et al.** *BMC Genomics,* 2014, vol. 15, 864
- **WITTIG, M. et al.** *Nucleic Acids Res.,* 2015, vol. 43 (11), e70
- **SAMBROOK, J. et al.** Molecular Cloning: A Laboratory Manual. Cold Spring Harbor Laboratory Press, 1998
- Standard Protocols on Next Generatin Sequencing. Experiment Medicine. YODOSHA CO., LTD, 2014
- **SZOLEK, A. et al.** OptiType: precision HLA typing from next-generation sequencing data. *Bioinformatics,* 2014, vol. 30, 3310-3316

- **HUANG, Y. et al.** HLA reporter: a tool for HLA typing from next generation sequencing data. *Genome Med,* 2015, vol. 7, 25
- **KAWAGUCHI et al.** HLA-HD: An accurate HLA typing algorithm for next-generation sequencing data. *Human Mutation,* 2017, vol. 38, 788-797
- **HOSOMICHI, K. et al.** Phase-defined complete sequencing of the HLA genes by next-generation sequencing. *BMC Genomics,* 2013, vol. 14, 355
- **EDGAR, R.C.** MUSCLE: multiple sequence alignment with high accuracy and high throughput. *Nucleic Acids Res.,* 2004, vol. 32, 1792-1797
- **UNTERGASSER, A. et al.** Primer3--new capabilities and interfaces. *Nucleic Acids Res.,* 2012, vol. 40, e115

SEQUENCE LISTING

<110>  Kyoto University

<120>  PCR PRIMER SET FOR HLA GENE, AND SEQUENCING METHOD USING SAME

<130>  092686

<150>  JP 2017-024397
<151>  2017-02-13

<160>  29

<170>  PatentIn version 3.5

<210>  1
<211>  24
<212>  DNA
<213>  Artificial Sequence

<220>
<223>  HLA-A forward primer

<400>  1
tttccagaga agccaatcag tgtc                                          24


<210>  2
<211>  30
<212>  DNA
<213>  Artificial Sequence

<220>
<223>  HLA-A reverse primer

<400>  2
acagacacat gcaggtgcct ttgcagaaac                                    30


<210>  3
<211>  26
<212>  DNA
<213>  Artificial Sequence

<220>
<223>  HLA-B forward primer

<400>  3
gtcgggtcct tcttccagga tactcg                                        26


<210>  4
<211>  30
<212>  DNA
<213>  Artificial Sequence

<220>
<223>  HLA-B reverse primer

<400>  4
tcttattttc agtagggaag taagaagttg                                    30

```
<210>   5
<211>   26
<212>   DNA
<213>   Artificial Sequence

<220>
<223>   HLA-C forward primer

<400>   5
gtcgggtcct tcttcctgaa tactca                                    26


<210>   6
<211>   30
<212>   DNA
<213>   Artificial Sequence

<220>
<223>   HLA-C reverse primer

<400>   6
ccacctcctc acattatgct aacaggaacg                                30


<210>   7
<211>   20
<212>   DNA
<213>   Artificial Sequence

<220>
<223>   HLA-DRB1 forward primer (set 1)

<400>   7
ccctccatct cctttactcc                                           20


<210>   8
<211>   20
<212>   DNA
<213>   Artificial Sequence

<220>
<223>   HLA-DRB1 reverse primer (set 1)

<400>   8
tgcctcccct cccacaacag                                           20


<210>   9
<211>   21
<212>   DNA
<213>   Artificial Sequence

<220>
<223>   HLA-DRB1 forward primer (set 2)

<400>   9
aggaggacct gtgaaccaga g                                         21


<210>   10
<211>   33
<212>   DNA
```

<213> Artificial Sequence

<220>
<223> HLA-DRB1 reverse primer (set 2)

<400> 10
tttgagaaac atttaataat gtaatgtgtt tgt                33

<210> 11
<211> 28
<212> DNA
<213> Artificial Sequence

<220>
<223> HLA-DQB1 forward primer

<400> 11
gtccttcagc tccagtgctg attggttc                      28

<210> 12
<211> 23
<212> DNA
<213> Artificial Sequence

<220>
<223> HLA-DQB1 reverse primer

<400> 12
gatgaaagga gatgacctgg tgg                           23

<210> 13
<211> 30
<212> DNA
<213> Artificial Sequence

<220>
<223> HLA-DPB1 forward primer (set 1)

<400> 13
cttatctgac tggttaaaat gagtatcact                    30

<210> 14
<211> 27
<212> DNA
<213> Artificial Sequence

<220>
<223> HLA-DPB1 reverse primer (set 1)

<400> 14
gaacccaaga caagtaaaat ccatctc                       27

<210> 15
<211> 30
<212> DNA
<213> Artificial Sequence

<220>

<223> HLA-DPB1 forward primer (set 2)

<400> 15
cagagtcttt cttataccaa agttgaagaa                    30

<210> 16
<211> 30
<212> DNA
<213> Artificial Sequence

<220>
<223> HLA-DPB1 reverse primer (set 2)

<400> 16
gaggatttca taatgatctc gatttgaaga                    30

<210> 17
<211> 24
<212> DNA
<213> Artificial Sequence

<220>
<223> HLA-A forward primer

<400> 17
atcctggata ctcacgacgc ggac                          24

<210> 18
<211> 25
<212> DNA
<213> Artificial Sequence

<220>
<223> HLA-A reverse primer

<400> 18
catcaacctc tcatggcaag aattt                         25

<210> 19
<211> 27
<212> DNA
<213> Artificial Sequence

<220>
<223> HLA-B forward primer

<400> 19
aggtgaatgg ctctgaaaat ttgtctc                       27

<210> 20
<211> 30
<212> DNA
<213> Artificial Sequence

<220>
<223> HLA-B reverse primer

<400> 20

agagtttaat tgtaatgctg ttttgacaca                                              30


<210>  21
<211>  24
<212>  DNA
<213>  Artificial Sequence

<220>
<223>  HLA-C forward primer

<400>  21
ggccgcctgt acttttctca gcag                                                    24


<210>  22
<211>  26
<212>  DNA
<213>  Artificial Sequence

<220>
<223>  HLA-C reverse primer

<400>  22
ccatggtgag tttccctgta caagag                                                  26


<210>  23
<211>  28
<212>  DNA
<213>  Artificial Sequence

<220>
<223>  HLA-DRB1 forward primer

<400>  23
tgattgactt gctggctggt ttctcatc                                                28


<210>  24
<211>  29
<212>  DNA
<213>  Artificial Sequence

<220>
<223>  HLA-DRB1 reverse primer

<400>  24
gcatccacag aatcacattt tctagtgtt                                               29


<210>  25
<211>  29
<212>  DNA
<213>  Artificial Sequence

<220>
<223>  HLA-DRB1 reverse primer

<400>  25
gcatccacag aatcacattt tccagtatt                                               29

```
<210>  26
<211>  26
<212>  DNA
<213>  Artificial Sequence

<220>
<223>  HLA-DQB1 forward primer

<400>  26
tcatgtgctt ctcttgagca gtctga                                      26


<210>  27
<211>  24
<212>  DNA
<213>  Artificial Sequence

<220>
<223>  HLA-DQB1 reverse primer

<400>  27
tgtgacagca attttctctc ccct                                        24


<210>  28
<211>  29
<212>  DNA
<213>  Artificial Sequence

<220>
<223>  HLA-DPB1 forward primer

<400>  28
tggtccaaca ggatcacatt tataagtgt                                   29


<210>  29
<211>  27
<212>  DNA
<213>  Artificial Sequence

<220>
<223>  HLA-DPB1 reverse primer

<400>  29
cccagtttgg atggtctctc agctctt                                     27
```